# EUROPÄISCHE PATENTSCHRIFT

(11) **EP 4 616 169 B1**
(45) Veröffentlichungstag und Bekanntmachung des Hinweises auf die Patenterteilung: **21.10.2026**
(21) Anmeldenummer: 23805521.4
(22) Anmeldetag: 09.11.2023
(51) Int. Cl.: G01N 1/31

(54) **VORRICHTUNG ZUM FÄRBEN VON AUF EINEM OBJEKTTRÄGER ANHAFTENDEN BIOLOGISCHEN PROBEN**
DEVICE FOR STAINING BIOLOGICAL SAMPLES ADHERING TO A SLIDE
DISPOSITIF DE COLORATION D'ÉCHANTILLONS BIOLOGIQUES ADHÉRANT À UNE LAME DE MICROSCOPE

(30) Priorität: 11.11.2022 DE 102022129863
(43) Veröffentlichungstag der Anmeldung: 17.09.2025
(73) Patentinhaber: Bio-Gram Diagnostics GmbH, 67550 Worms (DE)
(72) Erfinder: BLAKAJ, Petrit, 68542 Heddesheim (DE); KLUTH, Ian Sebastian, 64367 Mühltal (DE)
(74) Vertreter: Reiser & Partner Patentanwälte mbB
(86) Internationale Anmeldenummer: PCT/EP2023/081289
(87) Internationale Veröffentlichungsnummer: WO 2024/100187

(56) Entgegenhaltungen:
- WO-A1-2015/086534
- DE-A1- 10 041 229

## Beschreibung

Die Erfindung betrifft eine Vorrichtung zum Färben von auf einem Objektträger anhaftenden biologischen Proben, umfassend zumindest eine Färbekammer, zumindest einen Färbemittelbehälter, zumindest eine Färbemittelleitung, zumindest eine Färbemittelpumpe und eine Steuereinrichtung.

Eine derartige Vorrichtung ist aus der US 6,387,326 B1 bekannt. Im Bereich der Histologie, also der Untersuchung von biologischen Geweben, werden Färbemethoden eingesetzt, um Gewebestrukturen während einer optischen Untersuchung besser unterscheiden zu können. In diesem Zusammenhang sind verschiedene Färbemethoden bekannt, beispielsweise die HE-Färbung (Hämatoxylin-Eosin-Färbung). Bei dieser Färbung erscheinen beispielsweise Zellkerne des zu untersuchenden Gewebes blau-violett und Zytoplasma rosa. Intrazelluläre Substanzen färben sich ebenfalls an, beispielsweise Knorpel violett oder Fasern rosa. Ebenso werden Färbungen im Bereich der Hämatologie, also der Untersuchung von Blut und Knochenmark, eingesetzt.

Die zu untersuchenden biologischen Proben werden auf einen Objektträger aufgebracht und werden dann je nach Untersuchungsmethode mit verschiedenen Färbemitteln oder Färbeverfahren eingefärbt. Für das Erreichen einer optimalen Färbung ist dabei wichtig, dass die für die Färbung vorgesehenen Verfahren, insbesondere die Färbedauer und die Menge des aufgebrachten Färbemittels exakt eingehalten werden. Hierzu sind aus dem Stand der Technik Vorrichtungen bekannt, mit denen Objektträger mit darauf anhaftenden biologischen Proben automatisiert eingefärbt werden können. Dabei werden die Objektträger zumeist durch die Vorrichtung hindurchtransportiert und dabei einer Färbeprozedur unterzogen.

Dadurch, dass die Objektträger durch die Vorrichtung hindurch transportiert werden, ist es möglich, nacheinander eine Vielzahl von Objektträgern automatisiert zu behandeln. Nachteilig ist, dass die Vorrichtungen durch den Transportmechanismus und den auf den Transport abgestimmten Färbeprozess aufwendig und kostspielig sind. Des Weiteren ist der Einsatz derartiger Vorrichtungen erst bei größeren Stückzahlen einzufärbender Objektträger sinnvoll, bei kleineren Stückzahlen wäre die Vor- und Nachbereitung der Vorrichtung, beispielsweise die Reinigung und das Spülen im Verhältnis zu den eingefärbten Proben, zu groß.

Die WO 2015/086534 A1 beschreibt ein Verfahren zur Probenverarbeitung in einem automatisierten histologischen Färbesystem, bei dem ein Objektträger robotisch in ein Färbegerät des Systems eingeführt wird, anschließend für die Färbung automatisch Flüssigkeiten auf den Objektträger aufgebracht werden und der Objektträgerträger nach dem Färben robotisch wieder aus dem Färbegerät entnommen wird.

Die DE 100 41 229 A1 zeigt eine Vorrichtung zur Behandlung von Objekten, insbesondere von zytologischen oder histologischen Präparaten, mit mehreren in einem Gehäuse angeordneten Bearbeitungsstationen und einer Transporteinrichtung zum Verbringen von Objektträgern in die Bearbeitungsstationen hinein und aus den Bearbeitungsstationen heraus.

Der Erfindung liegt die Aufgabe zugrunde, eine Vorrichtung zum Färben von auf einem Objektträger anhaftenden biologischen Proben bereitzustellen, welche eine Färbung einer kleineren Stückzahl von Objektträgern bei gleichzeitig flexibler Anpassung der Färbemethode ermöglicht.

Diese Aufgabe wird mit den Merkmalen von Anspruch 1 gelöst. Auf vorteilhafte Ausgestaltungen nehmen die Unteransprüche Bezug.

Die erfindungsgemäße Vorrichtung zum Färben von auf einem Objektträger anhaftenden biologischen Proben umfasst zumindest eine Färbekammer mit einer Öffnung zur ortsfesten Aufnahme eines Objektträgers, zumindest einen Färbemittelbehälter, zumindest eine Färbemittelleitung, zumindest eine Färbemittelpumpe und eine Steuereinrichtung, wobei mittels der Färbemittelpumpe Färbemittel aus dem Färbemittelbehälter über die Färbemittelleitung in die Färbekammer förderbar sind.

Bei der erfindungsgemäßen Vorrichtung ist vorteilhaft, dass der Objektträger innerhalb der Vorrichtung an Ort und Stelle in der Färbekammer verbleibt und dort der Färbeprozedur unterzogen wird. Zum Färben wird das in einem Färbemittelbehälter bevorratete Färbemittel über eine Färbemittelpumpe in die Färbekammer gefördert, wobei der in der Färbekammer angeordnete Objektträger mit dem Färbemittel benetzt wird. Die Vorrichtung benötigt keinerlei bewegliche Elemente für den Transport von Objektträgern und ist daher besonders einfach und kostengünstig ausgebildet. Dementsprechend eignet sich die Vorrichtung insbesondere zum Färben einer kleineren Menge von Objektträgern.

Neben dem Färbemittelbehälter kann die Vorrichtung auch einen Spülmittelbehälter und einen Auffangbehälter aufweisen. Nach Abschluss der Färbung kann ein Spülmittel aus dem Spülmittelbehälter auf den Objektträger aufgetragen werden, um den Objektträger zu spülen und überschüssiges Färbemittel zu entfernen. Die Spüllösung, zusammengesetzt aus Färbemittel und Spülflüssigkeit, kann in dem Auffangbehälter gesammelt werden.

Die Färbekammer ist in einem Färbekammergehäuse aufgenommen, wobei die Öffnung ebenfalls in das Färbekammergehäuse eingebracht ist. Bis auf die Öffnung ist das Färbekammergehäuse vorzugsweise allseitig geschlossen und weist neben der Öffnung lediglich Zulauföffnungen und Ablauföffnungen für Färbemittel und Spüllösung auf. Dabei ist die Färbekammer kompakt ausgebildet und weist ein verhältnismäßig kleines Volumen auf und ermöglicht insgesamt eine platzsparende Ausgestaltung der Vorrichtung.

Das Färbekammergehäuse kann mehrere Färbekammern aufweisen, welche über Trennwände voneinander getrennt sind. Dabei können die Färbekammern nebeneinander und/oder übereinander angeordnet sein. Dabei ist jede Färbekammer zur Aufnahme eines Objektträgers ausgebildet, sodass jeder Objektträger separat in einer Färbekammer einfärbbar ist. Dadurch ist es prinzipiell möglich, mehrere Objektträger gleichzeitig einzufärben, wobei gleichzeitig verschiedene Färbemethoden durchführbar sind, um Objektträger wunschgemäß einfärben zu können.

Die Öffnung kann als Schlitz ausgebildet sein. Dabei orientiert sich die Breite und Höhe des Schlitzes an den Abmessungen des zu färbenden Objektträgers und ist vorzugsweise auf den Objektträger abgestimmt. Dadurch ist die Fläche der Öffnung klein und das Innere der Färbekammer ist weitgehend von der Umgebung abgetrennt, insbesondere kann verhindert werden, dass Färbemittel oder Spüllösung in der Färbekammer austrocknet. Des Weiteren kann eine Geruchsbelästigung oder Gesundheitsgefährdung durch in der Vorrichtung verdampfender Flüssigkeiten, beispielsweise von Färbereagenzien oder Spüllösungen, reduziert werden.

Der umlaufenden Kante der Öffnung kann eine Dichtung zugeordnet sein. Diese ist vorzugsweise so ausgebildet, dass die Dichtung an einem in der Färbekammer angeordneten Objektträger dichtend anliegt. Dadurch kann eine Geruchsbelästigung und/oder Gesundheitsgefährdung durch aus der Vorrichtung austretender Reagenzien nochmals reduziert werden. Die Dichtung kann als Lippendichtung ausgebildet sein, wobei die Lippe bei eingeschobenem Objektträger allseitig dichtend an dem Objektträger anliegt.

In die Färbekammer kann zumindest eine Zulauföffnung und zumindest eine Ablauföffnung eingebracht sein, wobei die Zulauföffnung mit der Färbemittelleitung verbunden ist. Über die Zulauföffnung wird Färbemittel in die Färbekammer eingebracht, welches dort den in der Färbekammer angeordneten Objektträger benetzt. Über die Ablauföffnung kann das Färbemittel aus der Färbekammer abfließen. Nach Abschluss der Färbung können die Objektträger mit einer Spüllösung gespült werden. Die Spüllösung kann über die Zulauföffnung eingebracht werden oder es kann eine weitere Zulauföffnung für die Zufuhr von Spüllösung vorgesehen sein. Die Spüllösung läuft über die Ablauföffnung ab.

Das Färbekammergehäuse kann ein plattenförmiges Unterteil aufweisen, welches mit einem plattenförmigen Oberteil verbunden ist, wobei in das Unterteil und/oder das Oberteil zur Ausgestaltung der Färbekammer zumindest eine Ausnehmung eingebracht ist. Ein derartiges Färbekammergehäuse ist einfach und kostengünstig herstellbar. Vorzugsweise ist das Färbekammergehäuse aus metallischem Werkstoff ausgebildet.

Zwischen Unterteil und Oberteil kann eine Dichtung, beispielsweise eine Flachdichtung, angeordnet sein. Die Zulauföffnung und/oder die Ablauföffnung mündet vorzugsweise in das Unterteil. Zum Färben der Objektträger werden die Objektträger so in der Färbekammer angeordnet, dass die an dem Objektträger anhaftenden biologischen Proben dem Unterteil zugewandt sind. Dabei kann der Objektträger flächig auf dem Unterteil beziehungsweise in der in das Unterteil eingebrachten Ausnehmung aufliegen. Wird über die Zulauföffnung ein Färbemittel in die Färbekammer eingebracht, benetzt das Färbemittel durch die Kapillarwirkung den Objektträger selbsttätig über die gesamte Fläche des Objektträgers, wobei überschüssiges Färbemittel über die Ablauföffnung abläuft.

Das Färbekammergehäuse kann geneigt in der Vorrichtung angeordnet sein. Dabei ist das Färbekammergehäuse vorzugsweise so angeordnet, dass die Färbekammern zur Horizontalen geneigt sind. Dabei ist das der Öffnung zugewandte Ende der Färbekammer höher als das der Öffnung gegenüberliegende Ende der Färbekammer. Dadurch kann einerseits verhindert werden, dass Färbemittel und/oder Spüllösung über die Öffnung nach außen gelangt. Andererseits ergibt sich eine besonders gute Verteilung des Färbemittels über die Oberfläche des Objektträgers, insbesondere wenn die Zulauföffnung im Bereich der Öffnung angeordnet ist und die Ablauföffnung dem der Öffnung gegenüberliegenden Ende der Färbekammer zugeordnet ist.

Die Ablauföffnung kann mit einer Absaugpumpe verbunden sein, wobei die Absaugpumpe über eine Ablaufleitung mit der Ablauföffnung verbunden sein kann. Über die Absaugpumpe kann Färbemittel und/oder Spüllösung aktiv aus der Färbekammer entfernt werden. Die Steuereinrichtung ist vorzugsweise eingerichtet, die Färbemittelpumpe zu beeinflussen, um Färbemittel aus dem Färbemittelbehälter in die Färbekammer zu fördern. Dabei beeinflusst die Steuereinrichtung die Färbemittelpumpe, um die auf dem Objektträger anhaftende biologische Probe zu färben.

Die Färbekammer kann einen Auflagebereich zur Lagerung des Objektträgers und eine durch den Auflagebereich begrenzte Vertiefung aufweisen. Der Auflagebereich ist vorzugsweise umlaufend im Randbereich der Färbekammer angeordnet und ermöglicht eine Lagerung eines Objektträgers auf dessen Randbereich. Die Vertiefung ist vorzugsweise napfförmig ausgebildet und der Boden der Vertiefung ist von einem auf den Auflagebereich aufgelegten Objektträger beabstandet. Die Vertiefung kann dabei so ausgebildet sein, dass sich in der Vertiefung eine Kapillarwirkung ausbildet, durch die sich Flüssigkeiten, wie Färbemittel und Spüllösungen, selbsttätig in der Vertiefung und an der Oberfläche des Objektträgers verteilen. Es ist aber auch denkbar, dass Flüssigkeiten durch Pumpen aktiv in die Vertiefung hinein- und hinausgefördert werden.

Die zumindest eine Zulauföffnung und die zumindest eine Ablauföffnung können in der Vertiefung angeordnet sein. Dadurch gelangen Flüssigkeiten besonders einfach und schnell in Kontakt mit dem Objektträger.

Mittels der Absaugpumpe kann ein Unterdruck in der Vertiefung erzeugbar sein, durch den der Objektträger in der Färbemittelkammer fixierbar ist. Dadurch ist der Objektträger in der Färbekammer fixiert. Flüssigkeiten können den Objektträger benetzen und treten dabei nicht aus der Färbekammer aus.

Die Färbemittelpumpe kann eingerichtet sein, eine gepulste Färbemittelströmung zu erzeugen. Dies kann insbesondere durch Peristaltikpumpen erfolgen, bei denen eine gepulste Strömung funktionsbedingt zumeist gegeben ist. Durch die gepulste Strömung erfolgt eine besonders schnelle und gründliche Benetzung des Objektträgers, so dass sich ein verbessertes Färbeergebnis ergibt und Prozesszeiten verkürzt werden können. Die Wirkung der gepulsten Strömung ist besonders gut, wenn in der Vertiefung bei aufgelegtem Objektträger Unterdruck herrscht und die gepulste Strömung in die Vertiefung eintritt und aus dieser wieder herausgeführt wird. Eine weitere Verbesserung ergibt sich, wenn die Vertiefung so ausgebildet ist, dass sich Flüssigkeiten auch durch Kapillarwirkung in der Vertiefung verteilen.

Die Einlauföffnung und die Ablauföffnung können mit Schnellverschlüssen versehen sein, welche ein schnelles und werkzeugloses Wechseln der Schläuche von Färbemittelleitung und Ablaufleitung ermöglichen.

In der Steuereinrichtung können auf verschiedene biologische Proben und/oder verschiedene Auswertemethoden abgestimmte Färbemittelarten und/oder Färbemittelmengen und/oder Färbemitteleinwirkdauern hinterlegt sein. Hierzu umfasst die Steuereinrichtung vorzugsweise einen Speicher, in welchem verschiedene Auswertemethoden und Färbemethoden sowie die dazugehörigen Prozeduren für die Färbung hinterlegt sind. Insbesondere ist denkbar, in der Steuereinrichtung Prozeduren für die folgenden Färbeverfahren zu hinterlegen:
Hämatoxylin-Eosin-Färbung (für die Histologie),
Pappenheim-Färbung (für die Hämatologie),
Wright-Giemsa-Färbung (für die Zytologie),
Gram-Färbung (für die Bakterienanalyse),
Ziehl-Neelsen-Färbung (für die Tuberkuloseanalyse) und
Papanicolau-Färbung (für die Gynäkologie).

Für die verschiedenen Färbemethoden kommen verschiedene Färbemittel und Spüllösungen zum Einsatz und die Einwirkdauer der Färbemittel und das Erfordernis einer Spülung und/oder Trocknung unterscheidet sich bei den verschiedenen Färbeverfahren. Die verschiedenen Prozeduren für die verschiedenen Färbeverfahren sind dafür in der Steuereinrichtung hinterlegt beziehungsweise hinterlegbar, sodass der Wechsel auf ein anderes Färbeverfahren besonders einfach ist. Darüber hinaus ist denkbar, einzelne Verfahrensparameter einer Färbeprozedur zu modifizieren, beispielsweise die zu den Zulauföffnungen geförderte Menge von Färbemittel und/oder Spülmittel oder auch eine mögliche Temperierung der Färbekammer. Dadurch können zur Erzielung besserer Färbeergebnisse einzelne Parameter angepasst werden.

Die Färbekammer kann temperierbar sein. Hierzu kann das Unterteil und/oder das Oberteil mit einem Heizelement ausgerüstet sein. Alternativ ist auch denkbar, ein Heizelement zwischen Unterteil und Oberteil anzuordnen. Das Heizelement ist vorzugsweise als elektrische Widerstandsheizung ausgebildet. Durch die Temperierung können Färbeprozesse beschleunigt werden, insbesondere weil Trocknungszeiten verkürzt werden können. Schon bei einer Erhöhung der Prozesstemperatur um 20 °C können die Prozesszeiten nahezu verdoppelt werden. Ein weiterer Vorteil ist auch, dass durch eine Temperierung besonders gleichbleibende Prozessbedingungen sichergestellt werden können.

Die Steuereinrichtung kann eingerichtet sein, durchgeführte Färbeverfahren zu erfassen und diese zu speichern. Dadurch kann im Nachhinein nachverfolgt werden, mit welchem Verfahren und mit welchen Prozessparametern Objektträger eingefärbt wurden. Dabei ist insbesondere auch denkbar, dass gespeicherte Färbungen über eine Schnittstelle an ein externes Speichermedium ausgegeben werden.

Der Steuereinrichtung ist vorzugsweise eine Eingabeeinrichtung zugeordnet. Vorzugsweise ist die Eingabeeinrichtung als Touch-Display ausgebildet. Über die Eingabeeinrichtung können besonders einfach Färbemittelverfahren ausgewählt und die zu einzelnen Verfahren gehörenden Prozessparameter modifiziert werden. Des Weiteren kann die Steuereinrichtung eine Schnittstelle aufweisen, über die alternative Färbeprozeduren in die Steuereinrichtung eingelesen werden können.

Einige Ausgestaltungen der erfindungsgemäßen Vorrichtungen werden nachfolgend anhand der Figuren näher erläutert. Diese zeigen, jeweils schematisch:
Fig. 1 eine Vorrichtung mit geöffneter Einschubvorrichtung;
Fig. 2 die Vorrichtung gemäß Fig. 1 mit geschlossener Einschubvorrichtung;
Fig. 3 die Vorrichtung gemäß Fig. 1 und Fig. 2 geöffnet, ohne Eingabeeinrichtung;
Fig. 4 im Detail die Einschubvorrichtung in der Draufsicht;
Fig. 5 die Einschubvorrichtung in der Seitenansicht;
Fig. 6 im Detail das Färbekammergehäuse der Vorrichtung in der Untersicht;
Fig. 7 das Färbekammergehäuse in der Draufsicht;
Fig. 8 ein Prozessschaubild.

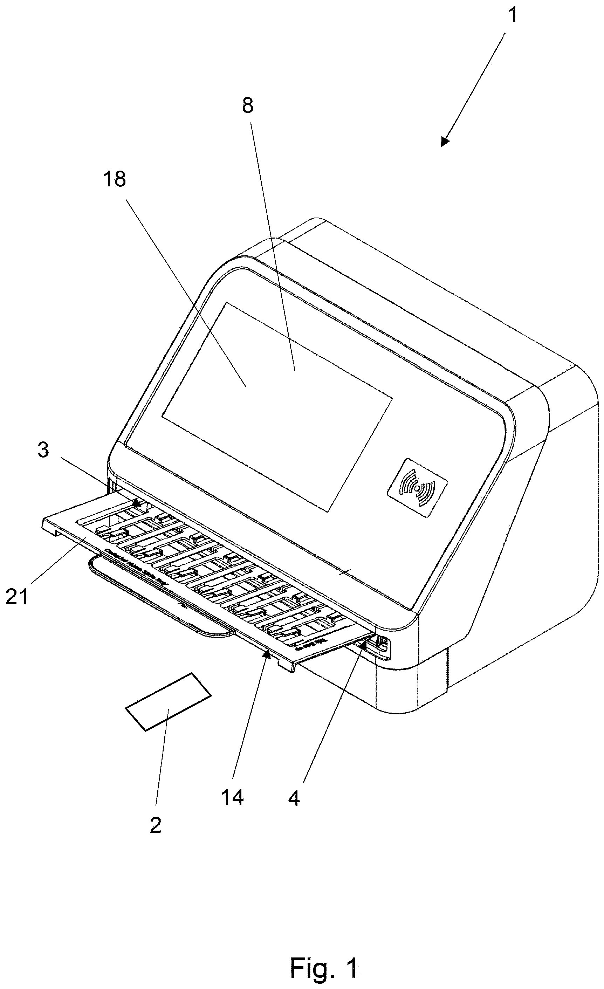

**Fig. 1** zeigt eine Vorrichtung 1 zum Färben von auf einem Objektträger 2 anhaftenden biologischen Proben. Die auf dem Objektträger 2 anhaftenden biologischen Proben werden in der Vorrichtung 1 mit einem oder mehreren Färbemitteln behandelt, um Strukturen der biologischen Proben unterschiedlich zu färben, sodass diese anschließend während einer optischen Untersuchung, beispielsweise im Rahmen einer Mikroskopie, besser unterschieden werden können.

Die Vorrichtung 1 umfasst mehrere Färbekammern 3, die jeweils mit einer Öffnung 4 zur ortsfesten Aufnahme eines Objektträgers 2 versehen sind. Die Färbekammern 3 sind in einem Färbekammergehäuse 9 aufgenommen, wobei die Öffnungen 4 in das Färbekammergehäuse 9 eingebracht sind. Bei der vorliegenden Ausgestaltung weist das Färbekammergehäuse 9 mehrere nebeneinander angeordnete Färbekammern 3 auf, welche über Trennwände 10 voneinander getrennt sind. Die Öffnungen 4 sind jeweils als Schlitz ausgebildet, wobei die Querschnittsfläche der Schlitze im Wesentlichen dem Querschnitt der untersuchenden Objektträger 2 entspricht. Jede Öffnung 4 ist mit einer Dichtung 14 in Form einer Lippendichtung versehen, wobei die Dichtung 14 jeweils der umlaufenden Kante einer Öffnung 4 zugeordnet ist und nach innen ragt, so dass die Dichtung 14 bei eingeschobenem Objektträger 2 allseitig dichtend an dem Objektträger 2 anliegt.

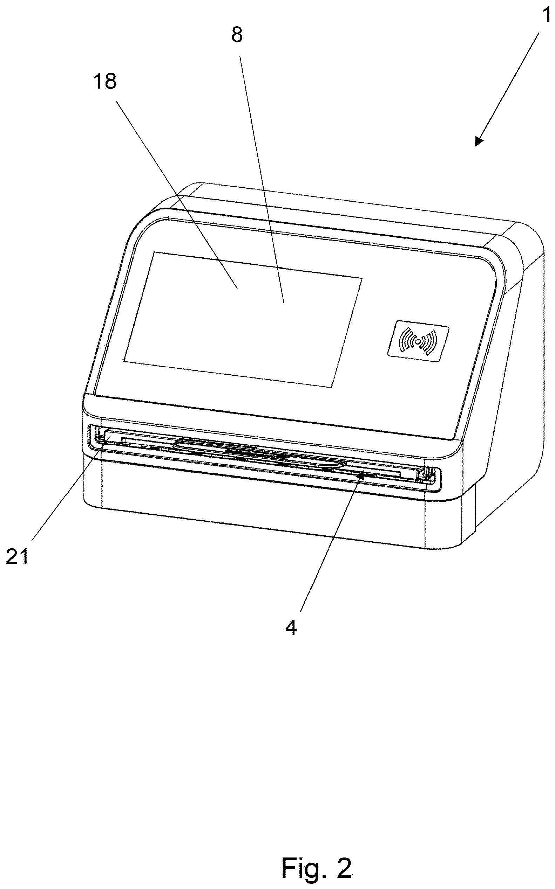

Bei der vorliegenden Ausgestaltung ist den Färbekammern 3 eine Einschubvorrichtung 21 in Form einer Schublade zugeordnet. **Fig. 1** zeigt die Einschubvorrichtung 21 in der geöffneten Position. **Fig. 2** zeigt die Vorrichtung 1 gemäß **Fig. 1** mit eingefahrener, geschlossener Einschubvorrichtung 21.

Die Einschubvorrichtung 21 weist Aufnahmen auf, in welchen mehrere Objektträger 2 gleichzeitig platziert werden können. Durch Einschieben der Einschubvorrichtung 21 in die Vorrichtung 1 werden alle Objektträger 2 gleichzeitig jeweils in eine separate Färbekammer 3 geführt. Das Einschieben der Einschubvorrichtung 21 erfolgt elektromotorisch über die mit dem Getriebe 23 der Einschubvorrichtung verbundenen Schrittmotoren 22. Die Einschubvorrichtung 21 ist mit Dichtungen 14 versehen, welche die Abdichtung der Färbekammern 3 zueinander und die Abdichtung der Färbekammern 3 in Richtung der Öffnung 4 gewährleisten.

Die Vorrichtung 1 umfasst ferner mehrere Färbemittelbehälter 5, Färbemittelleitungen 6 und mehrere Färbemittelpumpen 7 sowie eine Steuereinrichtung 8.

Die Steuereinrichtung 8 ist eingerichtet, die Färbemittelpumpen 7 zu beeinflussen, um die an dem Objektträger 2 anhaftenden biologischen Proben zu färben. Dabei sind in der Steuereinrichtung 8 auf verschiedene biologische Proben und/oder verschiedene Auswertemethoden abgestimmte Färbemittelarten, Farbemittemengen und Färbermitteleinwirkdauern hinterlegt. Dabei ist die Vorrichtung 1 eingerichtet, folgende Färbungen durchzuführen:
Hämatoxylin-Eosin-Färbung (für die Histologie),
Pappenheim-Färbung (für die Hämatologie),
Wright-Giemsa-Färbung (für die Zytologie),
Gram-Färbung (für die Bakterienanalyse),
Ziehl-Neelsen-Färbung (für die Tuberkuloseanalyse) und
Papanicolau-Färbung (für die Gynäkologie).

Der Steuereinrichtung 8 ist eine Eingabeeinrichtung 18 in Form eines Touch-Displays zugeordnet. Die Steuereinrichtung 8 ist ferner mit einem Speicher zur Speicherung der Färbeprozeduren ausgerüstet. In dem Speicher können des Weiteren durchgeführte Färbungen gespeichert werden. Die gespeicherten Färbungen können auch über eine Schnittstelle ausgegeben und beispielsweise an ein externes Speichermedium ausgegeben werden. In der Steuereinrichtung 8 sind für die oben beschriebenen Färbeverfahren vorbestimmte Prozeduren und Verfahrensparameter hinterlegt. Über die Eingabeeinrichtung können die Verfahrensparameter modifiziert und die Modifikation auch in der Steuereinrichtung 8 gespeichert werden. Dadurch ist es möglich, vorbestimmte Prozeduren zu ändern, beispielsweise um Färbeergebnisse zu ändern oder um die Vorrichtung 1 auf geänderte Färbemittel anpassen zu können. Auch angepasste Parameter können über die Schnittstelle ausgegeben werden.

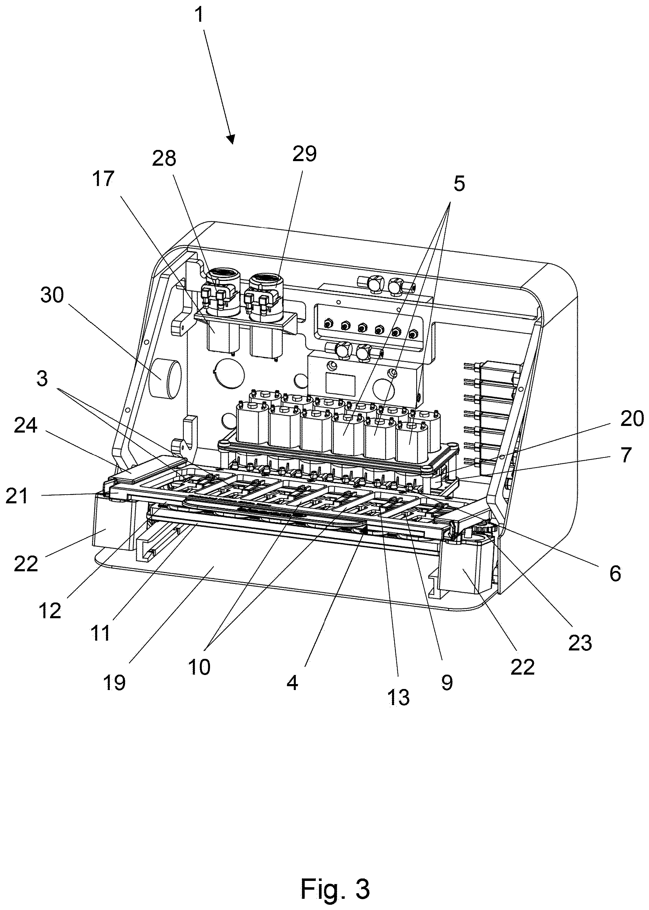

**Fig. 3** zeigt die Vorrichtung 1 gemäß **Fig. 1****,** wobei in dieser Darstellung die einen Deckel ausbildende Einheit aus Steuereinrichtung 8 und Eingabeeinrichtung 18 entfernt ist, so dass der Blick in das Innere der Vorrichtung 1 freigegeben ist. Die Einheit ist formschlüssig in der Vorrichtung 1 fixiert und kann für Wartungszwecke werkzeuglos entfernt werden. Dies ist besonders vorteilhaft, weil die aus flexiblen Schläuchen ausgebildeten Leitungen 6 einem Verschleiß unterliegen und schon aus den Gründen der Hygiene regelmäßig ausgetauscht werden müssen.

Bei der vorliegenden Ausgestaltung umfasst die Vorrichtung 1 ebenso viele Färbemittelpumpen 7 wie Färbekammern 3, sodass jeder Färbekammer 3 eine Färbemittelpumpe 7 zugeordnet ist. Mittels der Färbemittelpumpen 7 wird Färbemittel aus den Färbemittelbehältern 5 über die Färbemittelleitungen 6 in die Färbekammern 3 gefördert. Die Färbemittelpumpen 7 sind als Schlauchpumpen ausgebildet, wobei die Schlauchpumpen modular ausgebildet sind und mehrere Färbemittelpumpen 7 aneinander gekoppelt sind. Bei dieser Ausgestaltung ist jeder Färbemittelpumpe 7 ein Elektromotor 20 in Form eines Schrittmotors zugeordnet. In einer alternativen Ausgestaltung sind die Färbemittelpumpen 7 so gekoppelt, dass diese über einen einzigen Elektromotor 20 angesteuert werden. Die Färbemittelpumpen 7 sind eingerichtet, eine gepulste Färbemittelströmung zu erzeugen.

Das Färbekammergehäuse 9 ist geneigt in der Vorrichtung 1 angeordnet, der Neigungswinkel zur Horizontalen beträgt zehn Grad. Die Neigung ist dabei so ausgestaltet, dass sich das Färbekammergehäuse 9 in Richtung der Rückwand der Vorrichtung 1 absenkt.

Das nach dem Färbeprozess entfernte Färbemittel wird einem Flüssigkeitsreservoir 28 zugeführt. Benachbart zu dem Flüssigkeitsreservoir 28 ist ein Spülmittelbehältnis 29 zur Aufnahme von Spüllösung angeordnet.

An einer Seitenwand der Vorrichtung 1 ist ein Scanner 30 angeordnet. Der Scanner 30 ist bei der vorliegenden Ausgestaltung zum Auslesen von QR-Codes ausgerüstet und dient dem Einlesen von QR-Codes, die auf den Färbemittelbehältern 5 angebracht sind. Die eingelesenen Informationen werden an die Steuereinrichtung 8 weitergeleitet. Durch das Einlesen unmittelbar vor der Montage der Färbemittelbehälter 5 können Fehlbedienungen vermieden werden und die eingelesenen Informationen können für eine Programmwahl und für die Qualitätssicherung herangezogen werden.

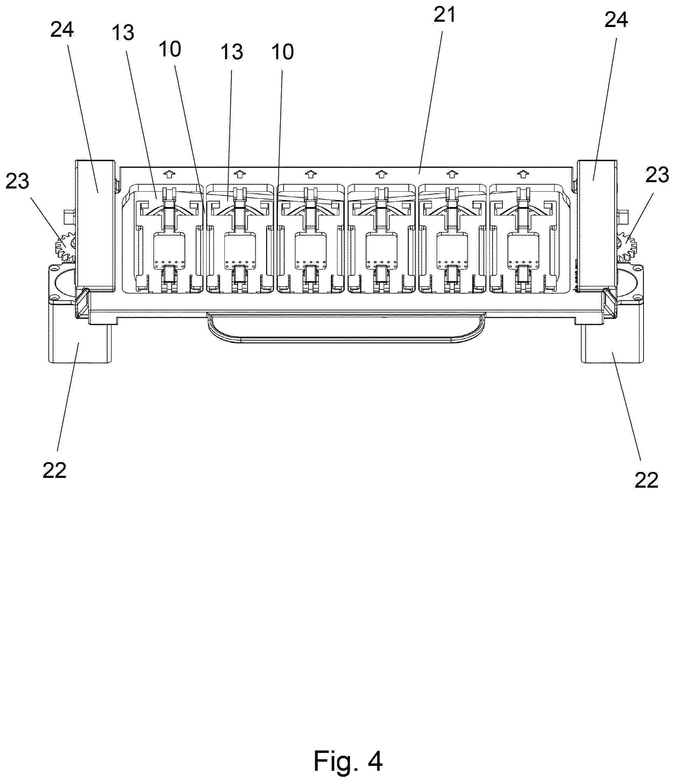

**Fig. 4** zeigt im Detail die Einschubvorrichtung 21 in der Draufsicht. In dieser Darstellung sind insbesondere die seitlich angeordneten Schrittmotoren 22 samt Getriebe 23 zu erkennen. Die Einschubvorrichtung 21 ist in seitlich angeordneten Führungsschienen 24 gelagert.

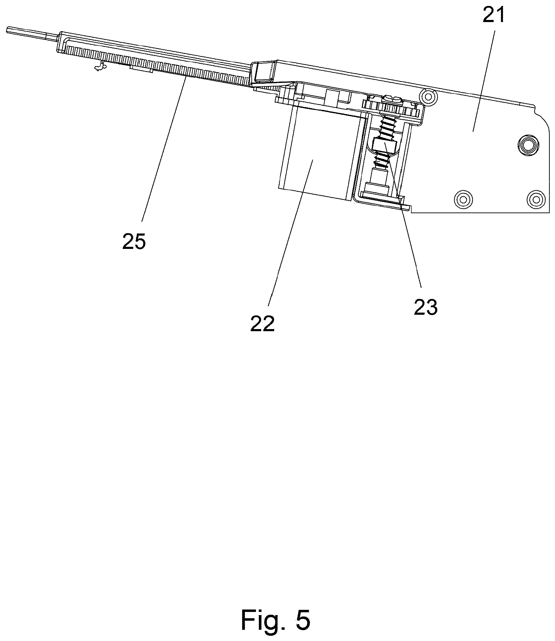

**Fig. 5** zeigt die Einschubvorrichtung 21 gemäß Fig. 4 in der Seitenansicht. In dieser Darstellung sind die Zahnstangen 25 zu erkennen, die seitlich an der Einschubvorrichtung angebracht sind und welche mit dem Getriebe 23 in Wirkverbindung stehen, um die Einschubvorrichtung 21 relativ zu dem Färbekammergehäuse 9 bewegen zu können.

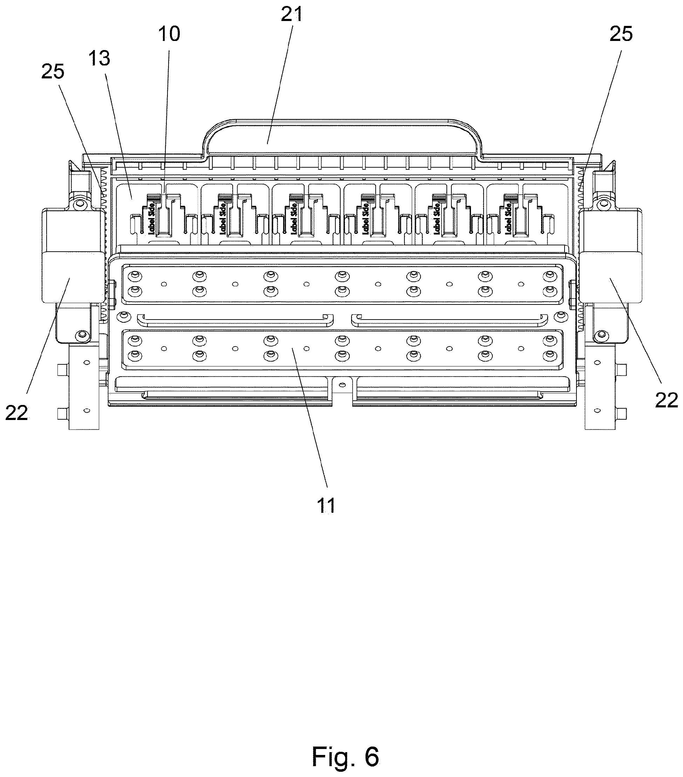

**Fig. 6** zeigt im Detail die Untersicht des Färbekammergehäuses 9 mit den darin angeordneten Färbekammern 3 und der Einschubvorrichtung 21. Bei der Darstellung sind insbesondere die in das Unterteil 11 mündenden Zulauföffnungen 15 und Ablauföffnungen 16, beziehungsweise die damit verbundenen Leitungen, zu erkennen.

Das Färbekammergehäuse 9 umfasst ein plattenförmiges Unterteil 11, welches mit einem plattenförmigen Oberteil 12 verbunden ist. Bei der vorliegenden Ausgestaltung sind die die Färbekammern 3 ausbildenden Ausnehmungen 13 in das Unterteil 11 eingebracht. Unterteil 11 und Oberteil 12 sind aus metallischem Werkstoff ausgebildet und über eine Schraubverbindung fest miteinander verbunden.

In die Färbekammern 3 sind jeweils Zulauföffnungen 15 und Ablauföffnungen 16 eingebracht, wobei die Zulauföffnungen 15 mit der Färbemittelleitung 6 verbunden sind. Die Ablauföffnungen 16 sind mit einem Auffangbehälter 19 verbunden. Zwischen den Ablauföffnungen 16 und dem Auffangbehälter 19 ist eine Absaugpumpe 17 angeordnet.

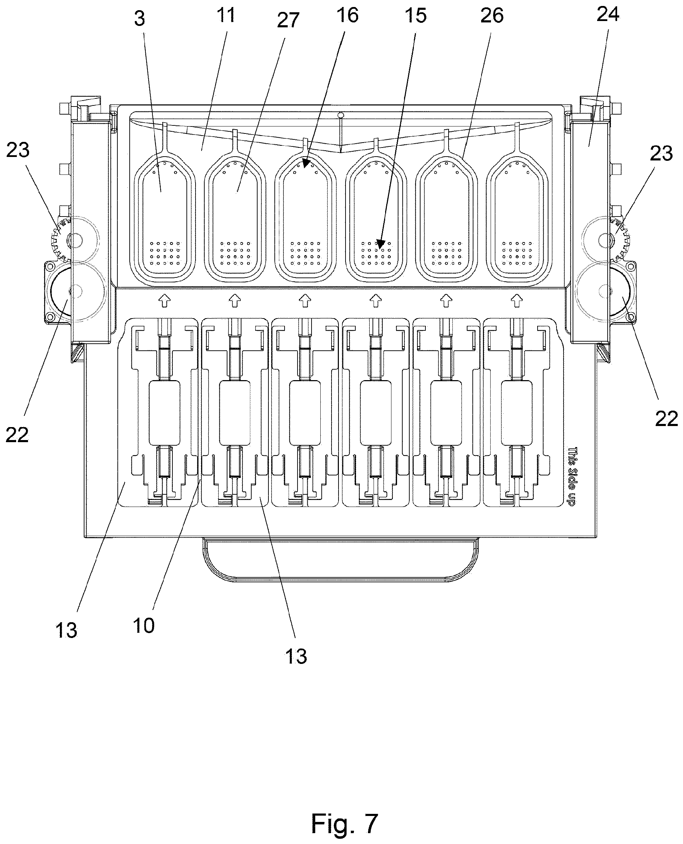

**Fig. 7** zeigt das Färbekammergehäuse 9 gemäß **Fig. 6** in der Draufsicht. In dieser Darstellung ist besonders gut zu erkennen, dass in das Unterteil 11 Vertiefungen 27 eingebracht sind, welche durch umlaufende Auflagebereiche 26 begrenzt sind. Die Zulauföffnungen 15 und die Ablauföffnungen 16 sind in der Vertiefung angeordnet, wobei die Zulauföffnungen einer Schmalseite und die Ablauföffnungen 16 der anderen Schmalseite zugeordnet sind. Mittels der Absaugpumpe 17 kann bei aufgelegtem Objektträger 2 ein Unterdruck in der Vertiefung 27 erzeugt werden, durch den der Objektträger 2 kraftschlüssig in der Färbekammer 3 fixiert ist.

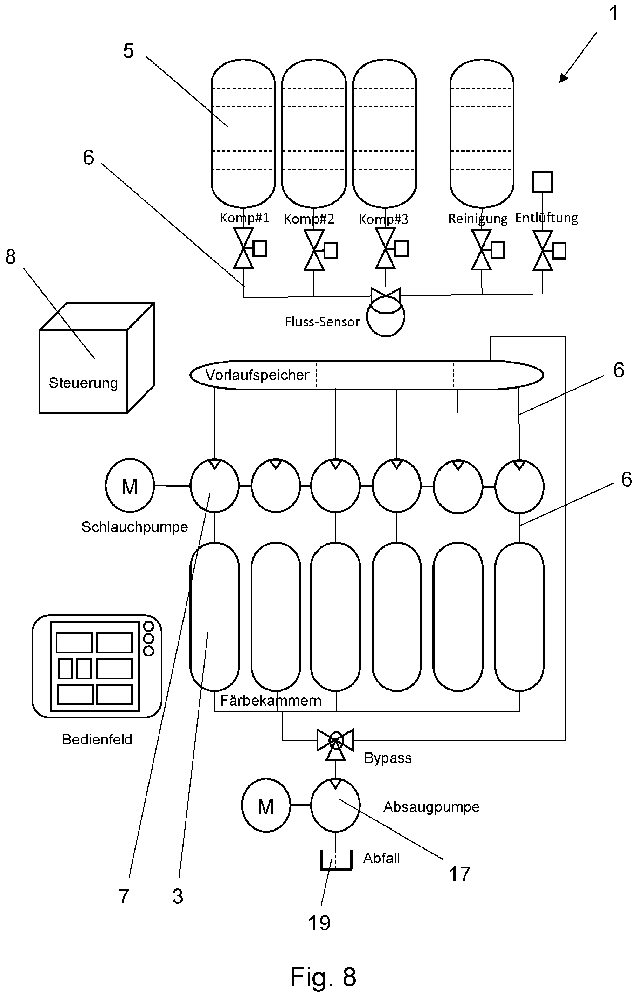

**Fig. 8** zeigt ein Prozessschaubild und eine schematische Darstellung der zuvor beschriebenen Komponenten der Vorrichtung 1.

## Patentansprüche

1. Vorrichtung (1) zum Färben von auf einem Objektträger (2) anhaftenden biologischen Proben, umfassend zumindest eine Färbekammer (3) mit einer Öffnung (4) zur ortsfesten Aufnahme eines Objektträgers (2), zumindest einen Färbemittelbehälter (5), zumindest eine Färbemittelleitung (6), zumindest eine Färbemittelpumpe (7) und eine Steuereinrichtung (8), wobei mittels der Färbemittelpumpe (7) Färbemittel aus dem Färbemittelbehälter (5) über die Färbemittelleitung (6) in die Färbekammer (3) förderbar ist, **dadurch gekennzeichnet, dass** die Färbekammer (3) in einem Färbekammergehäuse (9) aufgenommen ist, wobei die Öffnung (4) in das Färbekammergehäuse (9) eingebracht ist, wobei das Färbekammergehäuse (9) ein plattenförmiges Unterteil (11) aufweist, welches mit einem plattenförmigen Oberteil (12) verbunden ist, wobei in das Unterteil (11) und/oder das Oberteil (12) zur Ausgestaltung der Färbekammer (3) zumindest eine Ausnehmung (13) eingebracht ist.

2. Vorrichtung nach Anspruch 1, **dadurch gekennzeichnet, dass** das Färbekammergehäuse (9) mehrere Färbekammern (3) aufweist, welche über Trennwände (10) voneinander getrennt sind.

3. Vorrichtung nach Anspruch 2, **dadurch gekennzeichnet, dass** jeder Färbekammer (3) eine Färbemittelpumpe (7) zugeordnet ist.

4. Vorrichtung nach einem der Ansprüchen 1 bis 3, **dadurch gekennzeichnet,**
**dass** die Öffnung (4) als Schlitz ausgebildet ist.

5. Vorrichtung nach einem der Ansprüche 1 bis 4, **dadurch gekennzeichnet, dass** der umlaufenden Kante der Öffnung (4) eine Dichtung (14) zugeordnet ist.

6. Vorrichtung nach einem der Ansprüchen 1 bis 5, **dadurch gekennzeichnet, dass** in die Färbekammer (3) zumindest eine Zulauföffnung (15) und zumindest eine Ablauföffnung (16) eingebracht sind, wobei die Zulauföffnung (15) mit der Färbemittelleitung (6) verbunden ist.

7. Vorrichtung nach einem der Ansprüche 1 bis 6, **dadurch gekennzeichnet, dass** das Färbekammergehäuse (9) geneigt in der Vorrichtung (1) angeordnet ist.

8. Vorrichtung nach Anspruch 6 oder 7, **dadurch gekennzeichnet, dass** die Ablauföffnung (16) mit einer Absaugpumpe (17) verbunden ist.

9. Vorrichtung nach einem der Ansprüche 1 bis 8, **dadurch gekennzeichnet, dass** die Steuereinrichtung (8) die Färbemittelpumpe (7) beeinflusst, um Färbemittel aus dem Färbemittelbehälter (5) in die Färbemittelkammer (3) zu fördern.

10. Vorrichtung nach einem der Ansprüche 1 bis 9, **dadurch gekennzeichnet, dass** die Färbekammer (3) einen Auflagebereich (26) zur Lagerung des Objektträgers (2) und eine durch den Auflagebereich (26) begrenzte Vertiefung (27) aufweist.

11. Vorrichtung nach Anspruch 10, **dadurch gekennzeichnet, dass** die zumindest eine Zulauföffnung (15) und die zumindest eine Ablauföffnung (16) in der Vertiefung (27) angeordnet sind.

12. Vorrichtung nach Anspruch 11, **dadurch gekennzeichnet, dass** mittels der Absaugpumpe (17) ein Unterdruck in der Vertiefung erzeugbar ist, durch den der Objektträger (2) in der Färbemittelkammer (3) fixiert ist.

13. Vorrichtung nach einem der Ansprüche 3 bis 12, **dadurch gekennzeichnet, dass** die Färbemittelpumpe (7) eingerichtet ist, eine gepulste Färbemittelströmung zu erzeugen.

## Claims

1. Device (1) for staining biological samples which adhere to a slide (2), comprising at least one staining chamber (3) having an opening (4) for receiving a slide (2) in a stationary manner, at least one staining agent container (5), at least one staining agent line (6), at least one staining agent pump (7) and a control apparatus (8), with it being possible to convey staining agent from the staining agent container (5) into the staining chamber (3) via the staining agent line (6) by means of the staining agent pump (7), **characterized in that** the staining chamber (3) is received in a staining chamber housing (9), the opening (4) being introduced into the staining chamber housing (9), the staining chamber housing (9) having a planar lower part (11) which is connected to a planar upper part (12), with at least one recess (13) being introduced into the lower part (11) and/or the upper part (12) to form the staining chamber (3).

2. Device according to claim 1, **characterized in that** the staining chamber housing (9) has multiple staining chambers (3) which are separated from one another by partitions (10).

3. Device according to claim 2, **characterized in that** a staining agent pump (7) is associated with each staining chamber (3).

4. Device according to any of claims 1 to 3, **characterized in that** the opening (4) is designed as a slot.

5. Device according to any of claims 1 to 4, **characterized in that** a seal (14) is associated with the surrounding edge of the opening (4).

6. Device according to any of claims 1 to 5, **characterized in that** at least one inlet opening (15) and at least one outlet opening (16) are introduced into the staining chamber (3), the inlet opening (15) being connected to the staining agent line (6).

7. Device according to any of claims 1 to 6, **characterized in that** the staining chamber housing (9) is inclined in the device (1).

8. Device according to claim 6 or 7, **characterized in that** the outlet opening (16) is connected to a suction pump (17).

9. Device according to any of claims 1 to 8, **characterized in that** the control apparatus (8) influences the staining agent pump (7) to convey staining agent from the staining agent container (5) into the staining agent chamber (3).

10. Device according to any of claims 1 to 9, **characterized in that** the staining chamber (3) has a support region (26) for storing the slide (2) and a recess (27) which is delimited by the support region (26).

11. Device according to claim 10, **characterized in that** the at least one inlet opening (15) and the at least one outlet opening (16) are arranged in the recess (27).

12. Device according to claim 11, **characterized in that** by means of the suction pump (17), a vacuum can be generated in the recess, by which vacuum the slide (2) is secured in the staining agent chamber (3).

13. Device according to any of claims 3 to 12, **characterized in that** the staining agent pump (7) is designed to generate a pulsed staining agent flow.

## Revendications

1. Dispositif (1) destiné à la coloration d'échantillons biologiques fixés sur une lame porte-objet (2), comprenant au moins une chambre de coloration (3) comportant une ouverture (4) pour la réception de manière fixe d'une lame porte-objet (2), au moins un récipient pour colorant (5), au moins une conduite pour colorant (6), au moins une pompe à colorant (7) et un appareil de commande (8), dans lequel, au moyen de la pompe à colorant (7), le colorant peut être transporté depuis le récipient pour colorant (5) jusque dans la chambre de coloration (3) par l'intermédiaire de la conduite pour colorant (6), **caractérisé en ce que** la chambre de coloration (3) est reçue dans un boîtier pour chambre de coloration (9), dans lequel l'ouverture (4) est pratiquée dans le boîtier pour chambre de coloration (9), dans lequel le boîtier pour chambre de coloration (9) présente une partie inférieure (11) en forme de plaque qui est reliée à une partie supérieure (12) en forme de plaque, dans lequel au moins un évidement (13) est pratiqué dans la partie inférieure (11) et/ou dans la partie supérieure (12) pour aménager la chambre de coloration (3).

2. Dispositif selon la revendication 1, **caractérisé en ce que** le boîtier pour chambre de coloration (9) présente plusieurs chambres de coloration (3) qui sont séparées les unes des autres par l'intermédiaire de cloisons (10).

3. Dispositif selon la revendication 2, **caractérisé en ce qu'**une pompe à colorant (7) est associée à chaque chambre de coloration (3).

4. Dispositif selon l'une des revendications 1 à 3, **caractérisé en ce que** l'ouverture (4) est réalisée en tant que fente.

5. Dispositif selon l'une des revendications 1 à 4, **caractérisé en ce qu'**un joint d'étanchéité (14) est associé au bord périphérique de l'ouverture (4).

6. Dispositif selon l'une des revendications 1 à 5, **caractérisé en ce qu'**au moins une ouverture d'alimentation (15) et au moins une ouverture d'évacuation (16) sont pratiquées dans la chambre de coloration (3), dans lequel l'ouverture d'alimentation (15) est reliée à la conduite pour colorant (6).

7. Dispositif selon l'une des revendications 1 à 6, **caractérisé en ce que** le boîtier pour chambre de coloration (9) est disposé de manière à être incliné dans le dispositif (1).

8. Dispositif selon la revendication 6 ou 7, **caractérisé en ce que** l'ouverture d'évacuation (16) est reliée à une pompe d'aspiration (17).

9. Dispositif selon l'une des revendications 1 à 8, **caractérisé en ce que** l'appareil de commande (8) agit sur la pompe à colorant (7) afin de transporter le colorant depuis le récipient pour colorant (5) jusque dans la chambre pour colorant (3).

10. Dispositif selon l'une des revendications 1 à 9, **caractérisé en ce que** la chambre de coloration (3) présente une zone de soutien (26) pour le soutien de la lame porte-objet (2) et un renfoncement (27) délimité par la zone de soutien (26).

11. Dispositif selon la revendication 10, **caractérisé en ce que** l'au moins une ouverture d'alimentation (15) et l'au moins une ouverture d'évacuation (16) sont disposées dans le renfoncement (27).

12. Dispositif selon la revendication 11, **caractérisé en ce qu'**une dépression peut être générée dans le renfoncement à l'aide de la pompe d'aspiration (17), au moyen de laquelle dépression la lame porte-objet (2) est fixée dans la chambre pour colorant (3).

13. Dispositif selon l'une des revendications 3 à 12, **caractérisé en ce que** la pompe à colorant (7) est conçue pour générer un écoulement pulsé de colorant.
